# EUROPEAN PATENT APPLICATION

(11) **EP 4 260 895 A1**
(43) Date of publication of application: **18.10.2023**
(21) Application number: 21919158.2
(22) Date of filing: 28.12.2021
(51) Int. Cl.: A61M 25/09

(54) **CONVEYING GUIDE WIRE AND MANUFACTURING METHOD THEREFOR**

(30) Priority: 13.01.2021 CN 202110042074
(71) Applicant: MicroPort NeuroTech (Shanghai) Co., Ltd., Shanghai 201318 (CN)
(72) Inventor: CUI, Yafei, Shanghai 201318 (CN); LIU, Ziang, Shanghai 201318 (CN); ZHAO, Hanyi, Shanghai 201318 (CN); QIAO, Haiying, Shanghai 201318 (CN); TIAN, Hao, Shanghai 201318 (CN)
(74) Representative: Marks & Clerk LLP
(86) International application number: PCT/CN2021/142229
(87) International publication number: WO 2022/151971

(57) **Abstract**

A delivery guidewire (100), comprising: a core wire (110) comprising a first segment (111), a second segment (112) and a third segment (113), which are arranged in sequence and have diameters decreasing in the direction from a distal end to a proximal end of the delivery guidewire (100); a metal ring (130) at least partially disposed at a proximal end of the first segment (111); and a spring (120) at least partially disposed over the outer side of the second segment (112). A distal end of the spring (120) abuts against a proximal end of the metal ring (130), and a distal section of the spring (120) has a diameter smaller than a diameter of a proximal section thereof. The diameter of the distal section of the spring (120) that is smaller than the diameter of the proximal section of the spring (120) results in a smaller clearance between the distal section of the spring (120) and the core wire (110), which enables greater concentricity of the distal section of the spring (120) with the core wire (110). Also provided is a method of making a delivery guidewire (100).

## Description

### TECHNICAL FIELD

The present invention relates to the field of medical devices and, in particular, to a delivery guidewire and a method of making the delivery guidewire.

### BACKGROUND

Minimally invasive neurointerventional surgery provides a treatment for vascular aneurysms. It usually involves implanting a vascular implant, such as a stent, a coil or aneurysm occlusion device at a lesion site. Such implantation requires the use of a delivery guidewire and a catheter.

Existing delivery guidewires include a core wire acting as a key delivery component and a spring as a support member for the core wire and thereby ensuring desired delivery performance of the delivery guidewire. The spring should be maintained both concentric and stationary with respect to the core wire. Generally, the spring has a diameter across its distal section, which differs much from a diameter of the core wire. This makes it difficult to ensure concentricity of the distal section of the spring with the core wire and necessitates the use of a metal ring or a similar component for securing the spring relative to the core wire. This, however, in turn adds difficulty and cost to the manufacture of the delivery guidewire.

Therefore, there is a need for a novel delivery guidewire which overcomes at least the above disadvantages.

### SUMMARY OF THE INVENTION

It is an object of the present invention to provide a delivery guidewire which enables a distal section of the spring and a core wire to be more closely assembled, so that the distal section of the spring and the core wire are kept more concentric. The delivery guidewire can be more easily manufactured.

To this end, the present invention provides a delivery guidewire comprising:
a core wire comprising, arranged in sequence, a first segment, a second segment and a third segment, wherein diameters of the first, second and third segments gradually increase from a distal end to a proximal end thereof;
a metal ring at least partially disposed at a proximal end of the first segment; and
a spring at least partially sleeved over the outer side of the second segment, wherein the spring has a distal end abutting against a proximal end of the metal ring, and wherein an outer diameter of a distal section of the spring is smaller than an outer diameter of a proximal section of the spring.

Optionally, the distal section of the spring may have an inner diameter that is 0.01 -0.03 mm greater than a maximum outer diameter of the first segment.

Optionally, the metal ring may have a tapered proximal portion.

Optionally, the spring may have a varying-diameter section between its distal and proximal sections, the varying-diameter section having a diameter gradually increasing from the distal section to the proximal section.

Optionally, the metal ring may be provided with an opening at its proximal end, in which the distal section of the spring is received.

Optionally, an outer surface of the varying-diameter section and a proximal end face of the metal ring may form a recess, which is filled with a filler.

Optionally, the filler may be glue or a solder material.

Optionally, the opening may be flared so that both the distal and varying-diameter sections of the spring are received within the opening.

Optionally, the varying-diameter section of the spring may have three or more coil turns.

Optionally, the varying-diameter section of the spring may have a partial coil turn.

Optionally, the varying-diameter section of the spring may have a partial coil turn that is smaller than 3/4 of a complete coil turn, wherein detachment strength of the spring, the metal ring and the core wire ranges from 9 N to 10.4 N.

The present invention also provides a method of making the delivery guidewire as defined above, which includes:
providing the spring;
inserting the first segment of the core wire through at least part of the spring and sleeving the at least part of the spring over the second segment of the core wire; and
providing the metal ring, and inserting the first segment through at least part of the metal ring and sleeving the at least part of the metal ring over the proximal end of the first segment, so that the proximal end face of the metal ring abuts against the distal end of the spring.

Optionally, the metal ring may be provided with an opening at its proximal end, wherein after inserting the first segment through at least part of the spring and sleeving the at least part of the spring over the second segment, the distal section of the spring is received within the opening.

Optionally, the spring may have a varying-diameter section between its proximal and distal sections, wherein the opening is flared, and wherein after inserting the first segment through at least part of the spring and sleeving the at least part of the spring over the second segment, both the distal and varying-diameter sections of the spring are received within the opening.

In the delivery guidewire of the present invention, the diameter of the distal section of the spring that is smaller than the diameter of the proximal section of the spring results in a smaller clearance between the distal section of the spring and the core wire, which enables greater concentricity of the distal section of the spring with the core wire.

Moreover, the metal ring may be provided with an opening at its proximal end, in which the distal section of the spring is received. Connecting the spring, the metal ring and the core wire in this way enables enhanced detachment strength of them, which can reach as high as 6.1-10.4 N.

Additionally, the opening in the metal ring may be flared, and a varying-diameter section of the spring may be accommodated within the opening so that the joint of the spring and the core wire is located within the metal ring. In this way, the spring and the core wire can engage across a shorter length, enabling the delivery guidewire to have a shorter stiff portion. This imparts greater ability of the delivery guidewire to pass through bends at the same diameter.

Further, as a result of the varying-diameter section of the spring being received in place in the flared opening of the metal ring, the joint of the spring and the metal ring has a smooth circumference, which avoids the delivery guidewire from being stuck at an end face or a middle location of the microcatheter during its insertion or removal therein or therefrom.

Furthermore, the corresponding method makes it unnecessary to weld a distal end surface of a spring to a proximal end face of a metal ring and thereby enables the delivery guidewire to be made more easily.

### BRIEF DESCRIPTION OF THE DRAWINGS

Fig. 1 schematically illustrates an existing delivery guidewire.
Fig. 2 is a partial cross-sectional view of the existing delivery guidewire.
Fig. 3 is a diagram depicting a stent delivery system according to a first embodiment of the present invention in a first configuration.
Fig. 4 is a diagram depicting the stent delivery system according to the first embodiment of the present invention in a second configuration.
Fig. 5 is a diagram depicting the stent delivery system according to the first embodiment of the present invention in a third configuration.
Fig. 6 is a schematic illustration of a delivery guidewire according to the first embodiment of the present invention.
Fig. 7 is a schematic illustration of a core wire according to the first embodiment of the present invention.
Fig. 8 is a schematic illustration of a spring according to the first embodiment of the present invention.
Fig. 9 is a partial cross-sectional view of the delivery guidewire according to the first embodiment of the present invention.
Fig. 10 is a partial cross-sectional view of a delivery guidewire according to a second embodiment of the present invention.
Fig. 11 is a partial cross-sectional view of a delivery guidewire according to a third embodiment of the present invention.
Fig. 12 is a partial cross-sectional view of a delivery guidewire according to a fourth embodiment of the present invention.

In these figures,
100', a delivery guidewire; 110', a core wire; 120', a spring; 130', a metal ring;
100, a delivery guidewire; 110, a core wire; 111, a first segment; 112, a second segment; 113, a third segment; 114, a radiopaque tip section; 120, a spring; 121, a distal section of the spring; 122, a varying-diameter section of the spring; 123, a proximal section of the spring; 130, a metal ring; 140, a radiopaque ring;
200, a microcatheter;
300, a stent;
400, a blood vessel; and
410, a lesion site.

### DETAILED DESCRIPTION

Specific embodiments of the present invention will be described in greater detail with reference to the annexed schematic drawings. From the following description, advantages and features of the present invention will become more apparent. Note that the figures are provided in a very simplified form not necessarily drawn to exact scale and for the only purpose of facilitating easy and clear description of the embodiments disclosed herein.

As used herein, the term "proximal end" usually refers to an end of a medical device that is closer to an operator during normal operation thereof, while the term "distal end" usually refers to an end of the medical device that enters a patient's body first during normal operation thereof.

For the sake of concise and intuitive description, the present invention will be described below by way of some representative embodiments. Although numerous details have been set forth in the embodiments to provide an easier understanding of the present invention, it is apparent that the present invention can be implemented without being limited to these details. In order to avoid unnecessary obscuring of the invention, some embodiments are simply outlined but not elaborated. In this following, the term "comprising" is used in the sense of "including, but not limited to", and "according to" is used in the sense of "at least according to, but not limited to, only according to". Due to the characteristics of the Chinese language, for anything mentioned below, if it has not been specifically specified whether it is singular or plural, it is intended to mean that there may be either one or more such components, or at least one such component.

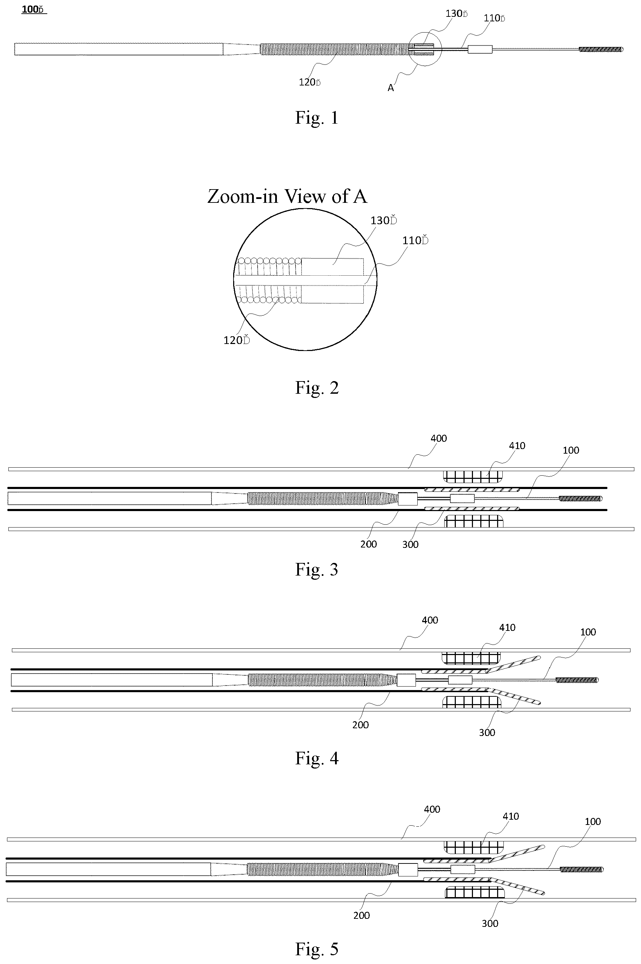

Fig. 1 is a schematic illustration of an existing delivery guidewire. Fig. 2 is a partial cross-sectional view of the existing delivery guidewire. As shown in Figs. 1 and 2, the delivery guidewire 100' includes a core wire 110', a metal ring 130' and a spring 120' and is used to deliver a stent. A distal end of the spring 120' is joined to a proximal end face of the metal ring 130' so as to be fixed in position relative to the core wire 110'. Since the spring 120' has a much larger diameter than the core wire 110', it is difficult to ensure concentricity of the distal end of the spring 120' with the core wire 110'.

Moreover, the distal end of the spring 120' is joined to the proximal end face of the metal ring 130' usually by welding, which, however, typically results in a lower connection strength at the joint, making the detachment strength of only 3-4 N of the spring 120', the metal ring 130' and the core wire 110' (the detachment strength is defined as the minimum force required to destroy any of the joints of the spring, the metal ring and the core wire).

In view of this, the present invention provides a delivery guidewire having greater distal concentricity of the spring with the core wire, and increased detachment strength of the spring, the metal ring and the core wire. Specific embodiments of the present invention will be described in detail below by way of examples.

### Embodiment 1

A lesion site of a human blood vessel may have stenosis or occlusion, which can be treated by dilating the stenotic or occluded segment of the blood vessel with a stent. Moreover, in order to keep the lesion site of the blood vessel segment dilated, the stent may be retained there. A stent intended for such retention is usually designed to be tubular. In a first embodiment of the present invention, a self-expanding or balloon-expandable stent is used. The self-expanding stent may be a braided stent having many helical filament elements that limit radial expansion. These filament elements may be made of a material, which exhibits a certain degree of stiffness while having certain degrees of flexibility and elasticity. The self-expanding stent is typically made of an alloy with shape memory or superelastic properties, such as Nitinol. Existing self-expanding stents can be used in the following two fields of therapeutic application. The first is neurointerventional treatment of intracranial vascular pathologies such as intracranial aneurysms and arteriovenous malformations. An intracranial aneurysm is a balloon-like bulge in the wall of a cerebral artery arising from abnormal local expansion of the arterial lumen typically caused by a congenital local defect in the arterial wall or a pressure increase in the arterial lumen, which is the number one cause of subarachnoid hemorrhage. The intracranial aneurysms can be well treated by neurointerventional stenting or coiling. The second field of application is the treatment of peripheral or intracranial vascular stenosis. Stenting of peripheral or intracranial blood vessels has become an important treatment option to treat the vascular stenosis.

In order to provide a further understanding of the proposed delivery guidewire, a process of delivering the stent, which is to be implanted to a stenotic area of a blood vessel, as an example, over the delivery guidewire will be briefly explained below with reference to the accompanying drawings. It will be appreciated that the delivery guidewire can also be used in the delivery of braided stents for aneurysmal treatment.

Fig. 3 is a diagram depicting a stent delivery system according to a first embodiment of the present invention in a first configuration. As shown in Fig. 3, the self-expanding stent 300 has been compressed to a smaller diameter over the delivery guidewire 100 and received in a microcatheter 200. The microcatheter 200 is inserted into a human body until the self-expanding stent 300 and the microcatheter 200 reach the lesion site 410 in the blood vessel 400. In other embodiments, the self-expanding stent 300 may be replaced with a balloon-expandable stent.

Fig. 4 is a diagram depicting the stent delivery system of this embodiment in a second configuration, and Fig. 5 is a diagram depicting the stent delivery system of this embodiment in a third configuration. As shown in Figs. 4 and 5, the delivery guidewire 100 is advanced, or the microcatheter 200 is retracted, so that self-expanding stent 300 carried on the delivery guidewire 100 is released from the microcatheter 200 at the lesion site 410. Due to its own expansion capability, the self-expanding stent 300 is able to expand by itself back to its original expanded shape, which allows the stent to firmly compress against and adhere to the wall of the blood vessel 400, thus maintaining the blood vessel 400 in a dilated state.

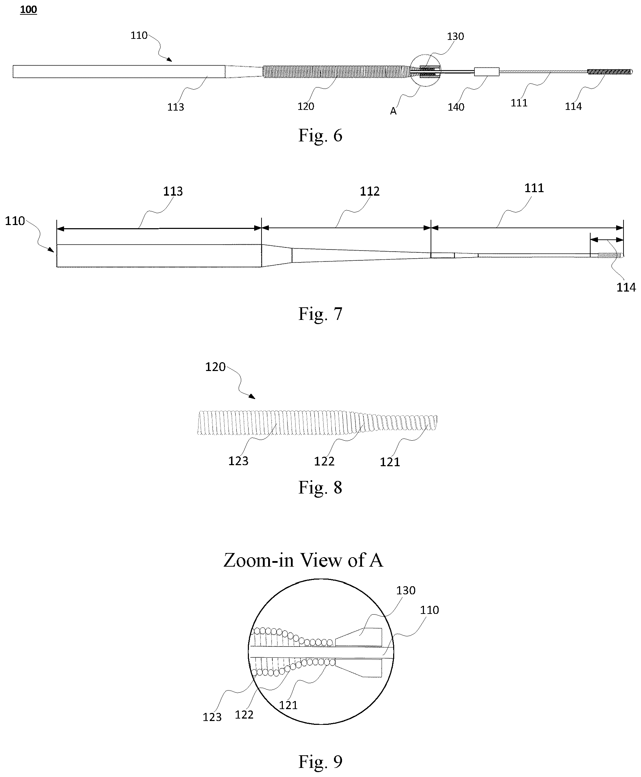

Fig. 6 is a schematic illustration of the delivery guidewire 100, and Fig. 7 schematically illustrates a core wire of according to this embodiment. As shown in Figs. 6 and 7, the delivery guidewire 100 includes a core wire 110, a metal ring 130 and a spring 120. The core wire 110 includes, arranged in sequence, a first segment 111, a second segment 112 and a third segment 113. Diameters of the first segment 111, the second segment 112 and the third segment 113 gradually increase from a distal end to a proximal end of the core wire. The core wire 110 may be made of stainless steel or another material chosen by a skill person based on his/her own experience, and the present invention is not limited thereto.

In a preferred embodiment, the third segment 113 has a length between 1140 mm and 1300 mm, the second segment 112 has a length between 240 mm and 420 mm, and the first segment 111 has a length between 25 mm and 135 mm. It would be recognized that the first segment 111 may further have a radiopaque tip section 114 at its distal end, which can provide, during a surgical procedure, a surgeon with an indication that the stent 300 is about to be pushed out of the microcatheter 200 (see Fig. 3). The radiopaque tip section 114 typically has a length between 5 mm and 15 mm.

With continued reference to Figs. 3 and 7, the third segment 113 of the core wire 110 is a proximal straight section, and its size is directly related to that of the associated microcatheter 200. In order to enable the delivery guidewire 100 to move within the microcatheter 200, the diameter of the third segment 113 should be smaller than that of the microcatheter 200, thereby leaving a clearance between an outer surface of the third segment 113 and an inner surface of the microcatheter 200, which can minimize resistance that the delivery guidewire 100 encounters when moving within the microcatheter 200. On the other hand, the diameter of the third segment 113 should be large enough to enable the delivery guidewire 100 to provide a required pushing force.

With continued reference to Figs. 3 and 7, the second segment 112 of the core wire 110 is a stent delivery section, which is critical component to transmission of a pushing force to a proximal end of the stent 300. When the delivery guidewire 100 is moving in the tortuous blood vessel 400, a pushing force may experience a loss during its transmission across the third segment 113 and the second segment 112 due to the elasticity of the blood vessel 400 and friction between the delivery guidewire 100 and the microcatheter 200. Moreover, as detailed below, the second segment 112 may be designed with a varying diameter, which leads to a smaller diameter and hence lower yield strength of the core wire 110 at a location closer to its distal end. When resistance that the second segment 112 of the core wire 110 encounters during its movement exceeds the yield strength, it may be bent into a serpentine shape, which will lead to even greater resistance. In view of this, the spring 120 is usually disposed over the second segment 112, in order to lower the chance of the second segment 112 being bent into a serpentine shape and thus allow the delivery guidewire 100 to encounter less resistance during its movement within the microcatheter 200.

With continued reference to Figs. 3, 6 and 7, the first segment 111 of the core wire 110 is a stent-bearing segment on which the stent 300 is crimped. The length of the first segment 111 may depend on a length, as well as other dimensions, of the stent 300. The radiopaque tip section 114 is provided at a distal section of the first segment 111. In other words, the first segment 111 generally comprises a proximal end secton of the radiopaque tip section 114. A portion of the first segment 111 adjacent the second segment 112 has at least a varying-diameter section and a straight section. The straight section is located at the proximal section of the first segment 111 and is joined to a distal end of the second segment 112 (i.e., the straight section is located on a proximal side of the varying-diameter section). In order to allow the stent 300 to be crimped as much as possible on the proximal end of the radiopaque tip section 114, the varying-diameter and straight sections should be as short as possible. Additionally, the first segment 111 is provided with a radiopaque ring 140. Usually, the proximal end of the stent 300 is located between the radiopaque ring 140 and the metal ring 130, and a distal end of the stent 300 is located between the radiopaque ring 140 and the radiopaque tip section 114. The straight section of the first segment 111 may have a diameter which is directly related to how the stent 300 is delivered and structured. Ways by which the stent 300 may be delivered may include, but are not limited to, a heat shrink tube and the radiopaque ring 140. It would be recognized that, in order to ensure joint strength and manufacturability of the second segment 112 and the first segment 111, the diameter of the straight section of the first segment 111 should not be below a lower limit. Moreover, in order to ensure distal compliance of the delivery guidewire 100, the diameter of the third segment 113 (i.e., the proximal straight segment) should not be above an upper limit. Accordingly, the diameter of the straight section of the first segment 111 typically lies between 0.08 mm and 0.12 mm, and it may be made of stainless steel or a nickel-titanium alloy.

With continued reference to Fig. 4, the core wire within the radiopaque tip section 114 may be either flat or round. In the latter case, theoretically, its diameter is desired to be as small as possible, as long as it can provide satisfactory tensile strength. However, limited by the state-of-the-art machining capability, a minimum possible value of the diameter that can be robustly achieved by grinding is about 0.04-0.06 mm. In order to reduce the risk of the core wire breaking at the radiopaque tip section 114 and ensure its consistent manufacture in batches, the diameter of the core wire at the section is typically 0.06 mm. In addition, in order to reduce stiffness of the radiopaque tip section 114, it may be flattened to a dimension typically of about 0.03 mm. The stiffness of the core wire within the radiopaque tip section 114can be additionally reduced using a shaping process. Most preferably, the stiffness of the core wire of the radiopaque tip section 114 is reduced by a combination of the flattening and shaping processes.

Fig. 8 is a schematic illustration of the spring 120 in this embodiment. As shown in Figs. 7 and 8, the spring 120 is sleeved over the second segment 112 and has a proximal section 123 and a distal section 121 with a diameter smaller than a diameter of the proximal section 123. A distal end of the spring 120 is joined to a proximal end of the metal ring 130, and a proximal end of the spring 120 may be joined to a proximal portion of the second segment 112. The proximal portion of the second segment 112 is a varying-diameter section. As the diameter of the distal section 121 is smaller than the diameter of the proximal section 123 of the spring, there is a smaller clearance between the distal section 121 of the spring and the core wire 110, which enables greater concentricity of the distal section 121 of this spring with the core wire 110. This can further lower the risk of the second segment 112 being bent into a serpentine shape and thus reduce resistance that the delivery guidewire 100 encounters during its movement within the microcatheter 200.

Additionally, an inner diameter of the distal section 121 of the spring is 0.01 -0.03 mm greater than a maximum outer diameter of the first segment 111. That is to say, an inner surface of the distal section 121 of the spring almost comes into contact with an outer surface of the first segment 111. This allows even greater concentricity of the distal section 121 of the spring with the core wire 110.

With continued reference to Figs. 8 and 9, the spring has a varying-diameter section 122 between its distal section 121 and proximal section 123. The proximal section 123 of the spring is a straight section, and the varying-diameter section 122 of the spring has a diameter gradually increasing in the direction from the distal section 121 to the proximal section 123. In some embodiments, the varying-diameter section 122 has three or more coil turns. In this way, the varying-diameter section 122 of the spring has a tapered shape, which facilitates movement of the delivery guidewire 100 within the microcatheter 200.

It would be recognized that the spring 120 may be made by coiling a metal wire. In some preferred embodiments, the coiling wire of the spring 120 has a diameter lying between 0.03 mm and 0.15 mm. It would be appreciated that the metal wire of the spring 120 may be so coiled that gaps are formed between adjacent turns or not, or that there are gaps between some adjacent turns while there are no gaps between the other turns.

Fig. 9 is a partial cross-sectional view of the delivery guidewire in this embodiment. As shown in Figs. 6 and 9, the metal ring 130 is sleeved over a proximal end of the first segment 111 (the metal ring 30 comprises a through opening with a size compatible with an outer diameter of the core wire 110 so that the core wire can be inserted therethrough). The distal end (face) of the spring 120 is joined to a proximal end face of the metal ring 130, and the metal ring 130 has a tapered proximal portion. It would be appreciated that the tapered shape of the proximal portion of the metal ring 130 can provide guidance for the metal ring 130, thereby mitigating collision of the metal ring 130 with the inner surface of the microcatheter 200. This can reduce resistance of the microcatheter 200 to the metal ring 130 during movement of the delivery guidewire 100 to a proximal end.

The present invention provides a corresponding method of making the delivery guidewire 100, which includes:
in step S 1, providing the spring 120 that includes the proximal section and the distal section 121 with a diameter smaller than a diameter of the proximal section;
in step S2, inserting the first segment 111 through at least a part of the spring 120 and sleeving the at least part of the spring 120 over the outer side of the second segment 112;
in step S3, providing the metal ring 130, and inserting the first segment 111 through at least a part of the metal ring 130 and sleeving the at least part of the metal ring 130 over the outer side of the proximal end of the first segment 111, so that the proximal end face of the metal ring 130 abuts against the distal end (face) of the spring 120.

This method may further include welding both the spring 120 and the metal ring 130 to the core wire 110. It is not necessary to weld the distal end (face) of the spring 120 to the proximal end face of the metal ring 130, making the assembly of the delivery guidewire 100 easier and reducing its manufacturing cost.

### Embodiment 2

In a second embodiment of the present invention, there is provided a delivery guidewire 100. In this following, only features unique to the second embodiment will be described below, while any common feature that it shares with the first embodiment will be omitted.

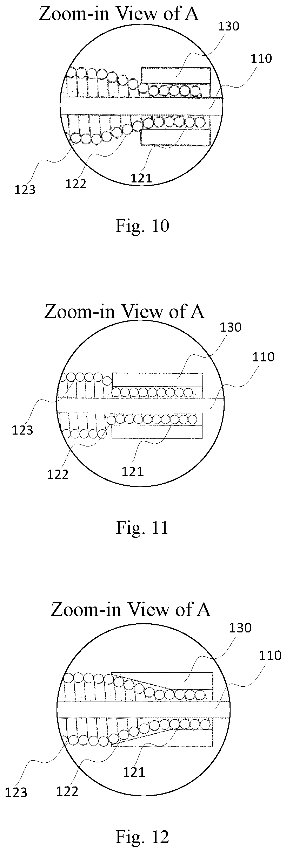

Fig. 10 is a partial cross-sectional view of the delivery guidewire 100 of Embodiment 2. As shown in Fig. 10, Embodiment 2 differs from the first embodiment in that: the proximal end of the metal ring 130 is provided with an opening, and the distal section 121 of the spring is received within opening of the metal ring 130 (i.e., compared with the first embodiment, the opening has a larger inner diameter at the proximal end of the metal ring in the second embodiment). Connecting the spring 120, the metal ring 130 and the core wire 110 in this way enables enhanced joint strength. The varying-diameter section 122 has three or more coil turns, and the detachment strength of the spring 120, the metal ring 130 and the core wire 110 may reach as high as 6.1-8.3 N.

The present invention provides a corresponding method of making the delivery guidewire 100, after inserting the first segment 111 through at least a part of the spring 120 and sleeving the at least part of the spring 120 over the outer side of the second segment 112, the distal section 121 of the spring is received within the opening. Moreover, the spring 120, the metal ring 130 and the core wire 110 are joined together by welding.

### Embodiment 3

In a third embodiment of the present invention, there is provided a delivery guidewire 100. Only features unique to the third embodiment will be described below, while any common feature that it shares with the first or second embodiment will be omitted.

Fig. 11 is a partial cross-sectional view of the delivery guidewire 100 according to Embodiment 3. As shown in Fig. 11, this embodiment differs from the second embodiment in that: the number of the coil turns in the varying-diameter section 122 of the spring is smaller than 1. This results in detachment strength of the spring 120, the metal ring 130 and the core wire 110 that is as high as 8-9.5 N. In some embodiments, the number of the coil turns of the varying-diameter section 122 is smaller than 3/4 of a complete coil turn. This allows a smaller gap to be left between the spring 120 and the metal ring 130 when the delivery guidewire 100 is assembled and can thereby further increase the detachment strength of the spring 120, the metal ring 130 and the core wire 110 to as high as 9-10.4 N.

It would be appreciated that an outer surface of the varying-diameter section 122 and the proximal end face of the metal ring 130 together form a recess, and during movement of the delivery guidewire 100, the recess will rub the inner surface of the microcatheter 200 and thus undesirably impede the movement of the delivery guidewire 100. In view of this, in the cases where the partial coil turn of the varying-diameter section 122 is smaller than 3/4 of a complete coil turn, the recess may be filled up with a filler so that the recess will not rub the inner surface of the microcatheter 200.

Optionally, the filler is glue or a solder material.

Additionally, the filler is glue exhibiting a certain degree of elasticity, which avoids the formation of any new recess as a result of bending of the delivery guidewire 100.

Referring to Fig. 10, in the second embodiment, since the varying-diameter section 122 of the spring has three or more coil turns, a rather large recess is formed. In contrast, Embodiment 3, the partial coil turn in the varying-diameter section 122 of the spring enables a smaller recess to be formed between the proximal end face of the metal ring 130 and the (outer surface) of the varying-diameter section 122 of the spring, which can facilitate movement of the delivery guidewire 100.

### Embodiment 4

In a fourth embodiment of the present invention, there is provided a delivery guidewire 100. Only features unique to this embodiment will be described below, while any common feature that it shares with the first, second or third embodiment will be omitted.

Fig. 12 is a partial cross-sectional view of the delivery guidewire in Embodiment 4. As shown in Fig. 12, Embodiment 4 differs from the second embodiment in that the proximal end of the opening of the metal ring 130 is flared (or tapered). A taper of the opening matches that of the varying-diameter section 122 in the spring, allowing the varying-diameter section 122 and the distal section 121 of the spring to be completely received in the opening (i.e., compared with the second embodiment, the opening has a different shape at the proximal end of the metal ring in the fourth embodiment). Unlike the second or third embodiment, since the outer diameter of the proximal section of the spring 120 is the same as the outer diameter of the metal ring 130, the joint of the spring 120 and the metal ring 130 has a smooth circumference without the recess that is presented at the Embodiments 2,3. This can avoid the delivery guidewire 100 from being stuck at an end face or a middle location of the microcatheter 200 during its insertion or removal therein or therefrom. This facilitates movement of the delivery guidewire 100.

In addition, the varying-diameter section of the spring 120 is received in the opening so that the joint of the spring 120 and the core wire 110 is located within the metal ring 130. At the same length of the metal ring 130, this enables the section of the spring 120 that fits against the core wire 110 (i.e., the distal section 121 thereof) to have a smaller length, compared with the second and third embodiments. This in turn enables the delivery guidewire 100 to have a shorter stiff portion and hence increased ability to pass through bends at the same diameter.

In summary, embodiments of the present invention provide a delivery guidewire including a core wire, a metal ring and a spring. The core wire has a first segment, a second segment and a third segment, which are arranged in sequence in the direction from a distal end to a proximal end of the delivery guidewire. Moreover, the core wire has a diameter segment-wise decreasing in the same direction. The metal ring is at least partially disposed at a proximal end of the first segment, and the spring is at least partially sleeved over the outer side of the second segment. A distal end of the spring abuts against a proximal end of the metal ring, and a distal section of the spring has a diameter smaller than a diameter of a proximal section of the spring. In the delivery guidewire of the present invention, the diameter of the distal section of the spring that is smaller than the diameter of the proximal section of the spring results in a smaller clearance between the distal section of the spring and the core wire, which enables greater concentricity of the distal section of the spring with the core wire. Moreover, the metal ring may comprise an opening at its proximal end, in which the distal section of the spring is received. Connecting the spring, the metal ring and the core wire in this way enables enhanced joint strength, and enables the detachment strength of them to reach as high as 6.1-10.4 N. Additionally, the opening in the metal ring may be flared, and a varying-diameter section of the spring may be accommodated within the opening so that the joint of the spring and the core wire is located within the metal ring. In this way, the spring and the core wire can engage across a shorter length, enabling the delivery guidewire to have a shorter stiff portion. This imparts greater ability of the delivery guidewire to pass through bends at the same diameter. Further, as a result of the varying-diameter section of the spring being received in the flared opening of the metal ring, the joint of the spring and the metal ring has a smooth circumference, which avoids the delivery guidewire from being stuck at an end face or a middle location of the microcatheter during its insertion or removal therein or therefrom. The present invention also provides a corresponding method of making a delivery guidewire, which makes it unnecessary to weld a distal end surface of a spring to a proximal end face of a metal ring and thereby enables the delivery guidewire to be made more easily.

Presented above are merely some preferred embodiments of the present invention, which do not limit the invention in any way. Changes in any forms made to the principles and teachings disclosed herein, including equivalents and modifications, by any person of ordinary skill in the art without departing from the scope of the invention are intended to fall within the scope of the invention.

## Claims

1. A delivery guidewire, comprising:
a core wire comprising, arranged in sequence, a first segment, a second segment and a third segment, wherein diameters of the first, second and third segments gradually increase from a distal end to a proximal end thereof;
a metal ring at least partially disposed at a proximal end of the first segment; and
a spring at least partially sleeved over an outer side of the second segment, wherein the spring has a distal end abutting against a proximal end of the metal ring, and wherein an outer diameter of a distal section of the spring is smaller than an outer diameter of a proximal section of the spring.

2. The delivery guidewire of claim 1, wherein the distal section of the spring has an inner diameter that is 0.01 -0.03 mm greater than a maximum outer diameter of the first segment.

3. The delivery guidewire of claim 1, wherein the metal ring has a tapered proximal portion.

4. The delivery guidewire of claim 1, wherein the spring has a varying-diameter section between the distal and proximal sections thereof, wherein the varying-diameter section has a diameter gradually increasing from the distal section to the proximal section.

5. The delivery guidewire of claim 4, wherein a proximal end of the metal ring is provided with an opening, and wherein the distal section of the spring is received in the opening.

6. The delivery guidewire of claim 5, wherein an outer surface of the varying-diameter section and a proximal end face of the metal ring form a recess, wherein the recess is filled with a filler.

7. The delivery guidewire of claim 6, wherein the filler is a glue or a solder material.

8. The delivery guidewire of claim 5, wherein the opening is flared so that each of the distal and varying-diameter sections of the spring is received within the opening.

9. The delivery guidewire of claim 5, wherein a number of coil turns of the varying-diameter section of the spring is greater than 3.

10. The delivery guidewire of claim 5, wherein a number of coil turns of the varying-diameter section of the spring is smaller than 1.

11. The delivery guidewire of claim 10, wherein the number of coil turns of the varying-diameter section of the spring is smaller than 3/4 of a complete coil turn, wherein a detachment strength of the spring, the metal ring and the core wire ranges from 9 N to 10.4 N.

12. A method of making the delivery guidewire of any one of claims 1 to 11, comprising:
providing a spring;
inserting a first segment of the core wire through at least part of the spring and sleeving the at least part of the spring over a second segment of the core wire; and
providing a metal ring, and inserting the first segment through at least part of the metal ring and sleeving the at least part of the metal ring over a proximal end of the first segment, so that a proximal end face of the metal ring abuts against a distal end of the spring.

13. The method of claim 12, wherein a proximal end of the metal ring is provided with an opening, wherein after inserting the first segment through the at least part of the spring and sleeving the at least part of the spring over the second segment, a distal section of the spring is received within the opening.

14. The method of claim 13, wherein the spring has a varying-diameter section between a proximal section and a distal section thereof, wherein the opening is flared, and wherein after inserting the first segment through the at least part of the spring and sleeving the at least part of the spring over the second segment, each of the distal section and varying-diameter section of the spring is received within the opening.
